# EUROPEAN PATENT APPLICATION

(11) **EP 4 491 134 A1**
(43) Date of publication of application: **15.01.2025**
(21) Application number: 23766521.1
(22) Date of filing: 20.02.2023
(51) Int. Cl.: A61B 17/02

(54) **SUCTION ATTACHMENT AND STABILIZER**

(30) Priority: 07.03.2022 JP 2022034568
(71) Applicant: Vital Corporation, Tokyo, 140-0002 (JP)
(72) Inventor: ICHINOSEKI, Masao, Tokyo 140-0002 (JP); AONUMA, Takashi, Tokyo 140-0002 (JP); TATEISHI, Tomoki, Tokyo 140-0002 (JP); SHIMIZU, Tatsuya, Tokyo 140-0002 (JP)
(74) Representative: Maiwald GmbH
(86) International application number: PCT/JP2023/006008
(87) International publication number: WO 2023/171342

(57) **Abstract**

To provide a suction attachment that can sufficiently suppress vibrations caused by a heartbeat during coronary artery bypass surgery relative to a contact part of the suction attachment and avoid a problem of breakage after coronary artery bypass surgery and a stabilizer equipped with the suction attachment. The suction attachment 50, which is attached to the stabilizer 100 that suppresses vibrations of the heart surface of a patient undergoing coronary artery bypass surgery, includes a flexible channel 60 that forms a suction path 61 in conjunction with the heart surface when in contact with the heart surface of the patient, and the flexible channel 60 has a C-shaped tunnel configuration that serves as a passageway for the graft vessel.

## Description

### [FIELD OF THE INVENTION]

The present invention relates to a suction attachment and a stabilizer, particularly for use in human and animal coronary artery bypass surgery.

### [BACKGROUND OF THE INVENTION]

Patent Document 1 discloses a hemostatic holding device for vascular anastomosis used for coronary artery bypass surgery, which is characterized that the hemostatic holding device has a suction cup body with a flexible channel capable of adhering to a heart wall surface while surrounding a coronary artery, which has a treatment opening at a central part of the suction cup body for exposing and treating the anastomosis position of the coronary artery and that the flexible channel is communicatively connected to an exhaust tube for expelling air to the outside. The flexible channel is disclosed to have a donut shape as shown in the claim 2 and FIG. 2 of Patent Document 1, and a horseshoe shape as shown in the claim 3 and FIG. 4 of the same.

### [PRIOR ARTS]

### [PATENT DOCUMENT]

[PATENT DOCUMENT 1] Specification of the Japanese Patent No. 3036686

### [Disclosure of the Invention]

### [Problems to be Resolved by the Invention]

However, in the hemostatic retention device described in Patent Document 1, when the flexible channel is shaped like a donut, a graft vessel is anastomosed to the heart surface located within the central processing opening, and therefore the flexible channel surrounds the graft vessel subsequently. Accordingly, thereafter, it becomes necessary to perform a rupture procedure on the flexible channel to create space for passing the graft vessel, followed by removing the flexible channel. (See the paragraph 0030 of Patent Document 1)

However, when rupturing the flexible channel, there is a potential risk of generating small fragments that are difficult to visually confirm. If these fragments remain around the heart, they could pose a problem by potentially injuring the heart post-surgery.

On the other hand, in the hemostatic retention device described in Patent Document 1, when the flexible channel is shaped like a horseshoe, although no rupture procedure is necessary, the vibration of the heart surface due to cardiac pulsation during surgery is transmitted into the processing opening through the relatively large release portion of the horseshoe shape, thereby hindering the stabilizing function from being fully effective.

Therefore, the present invention aims to provide a suction attachment and a stabilizer that can sufficiently suppress vibrations caused by cardiac pulsation during surgery at the contact portion of the suction attachment, while also avoiding postoperative issues related to rupture.

### [Means to Resolve the Problem(s)]

In order to resolve the above-described problems, the present invention provides a suction attachment attached to a stabilizer that suppresses vibrations of a heart surface of a patient undergoing a coronary artery bypass surgery, which comprises a flexible channel that forms a suction flow path together with the heart surface when the suction attachment is brought into contact with the heart surface of the patient, wherein the flexible channel is configured to have a C-shaped tunnel shape to serve as a passage of a graft vessel.

The suction attachment may further comprises a C-shaped flexible plate supporting a shape of the flexible channel, wherein a gap between one end and the other end of the flexible channel is aligned with a gap between one end and the other end of the flexible plate.

A width of the gap between one end and the other end of the flexible channel may be variable due to the deformation of the flexible channel.

The flexible channel may have a plurality of locking sections on an outer upper corner.

The suction flow path may have a relatively thick, straight cross-sectional inner inclined section and a relatively thin, curved cross-sectional inclined section.

The flexible channel may have a suction port connected to the suction flow path.

The stabilizer of the present invention is such that the suction attachment is connected to a main body of the stabilizer using a universal joint.

### [BRIEF EXPLANATION OF THE DRAWING(S)]

FIG. 1 is a perspective view showing the schematic appearance of the stabilizer 100 according to an embodiment of the present invention;
FIG. 2A is a right side view of the stabilizer 100 shown in FIG. 1;
FIG. 2B is a cross-sectional view taken along line A-A of the stabilizer 100 shown in FIG. 2A;
FIG. 2C is a left side view of the stabilizer 100 shown in FIG. 2A;
FIG. 2D is a right side view of the stabilizer 100 shown in FIG. 2A;
FIG. 3A is a perspective photograph of the suction attachment 50 shown such as in FIG. 1;
FIG. 3B is a CAD plan view of the suction attachment 50 shown in FIG. 3A;
FIG. 3C is a CAD front view of the suction attachment 50 shown in FIG. 3A;
FIG. 3D is a CAD right side view of the suction attachment 50 shown in Figure 3A;
FIG. 4A is a plan view of the flexible channel 60 shown in FIG. 3A;
FIG. 4B is a front view of the flexible channel 60 shown in FIG. 4A;
FIG. 4C is a cross-sectional view taken along line C-C of the flexible channel 60 shown in FIG. 4A; and
FIG. 4D is a cross-sectional view taken along line D-D of the flexible channel 60 shown in FIG. 4A.

### [Embodiment(s) of the Invention]

In the following sections, embodiments of the present invention will be explained with reference to the attached drawings.

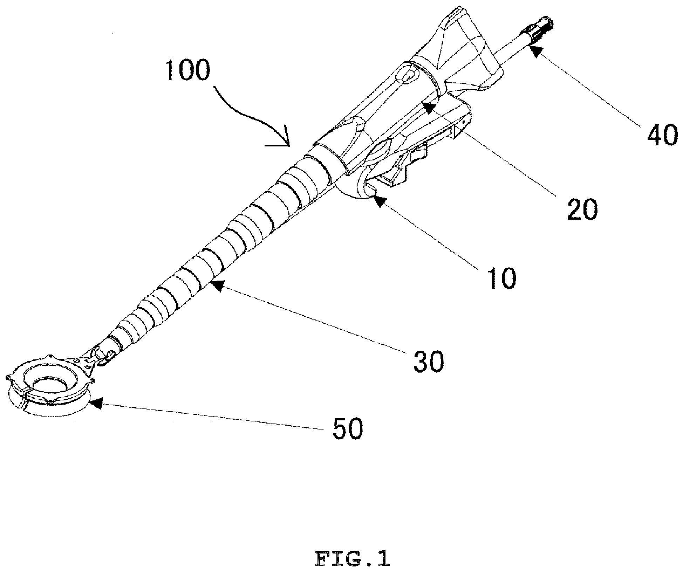

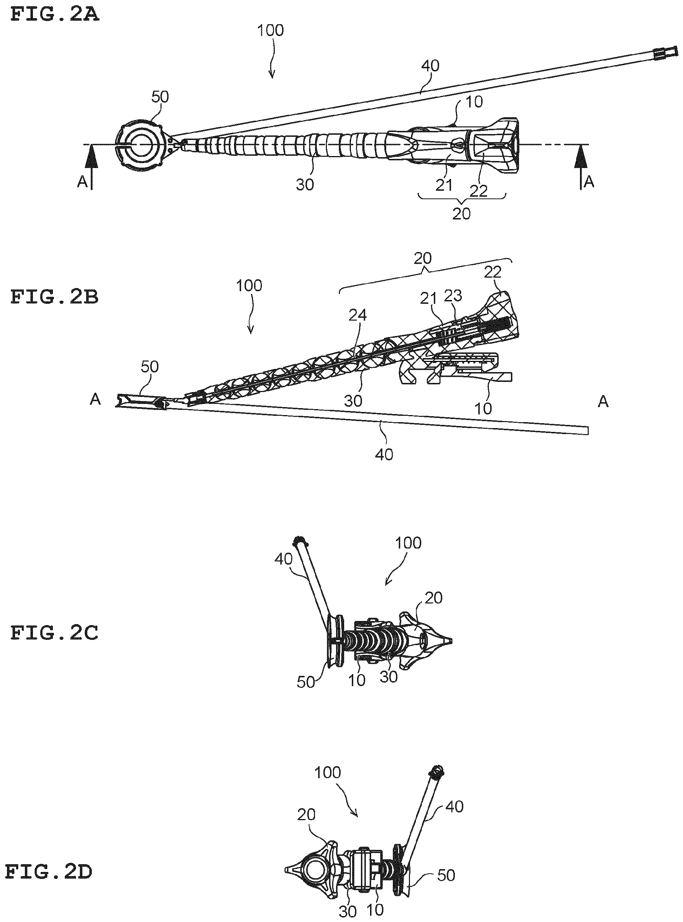

FIG. 1 is a perspective view illustrating a schematic appearance of the stabilizer 100 according to an embodiment of the present invention. FIG. 2A is a plan view of the stabilizer 100 shown in FIG. 1. FIG. 2B is a cross-sectional view taken along line A-A of FIG. 2A showing the stabilizer 100. FIG. 2C is a left side view of the stabilizer 100 shown in FIG. 2A. FIG. 2D is a right side view of the stabilizer 100 shown in FIG. 2A.

The stabilizer 100 shown in FIG. 1 and FIGS. 2A to 2D is designed to suppress vibrations on the heart surface of the patient during coronary artery bypass surgery. FIG. 1 and FIGS. 2A to 2D illustrate the following components, which will be described below: a clamp 10, a main body 20, an arm 30, a tube 40, and a suction attachment 50.

The clamp 10 is used to secure the stabilizer 100 to other surgical instruments, such as a chest retractor (not shown), in the vicinity of the operating table. The clamp 10 can achieve this fixation through any shape or mechanism, provided it does not interfere with the surgery. The clamp 10 is typically made of stainless steel, aluminum, titanium, or resin, as commonly used for medical instruments.

The main body 20, as shown in FIG. 1, is connected to the clamp 10 and the arm 30. As depicted in FIG. 2B, the main body 20 includes a grip portion 21 for the operator, such as a doctor, to hold, a handle 22 for fixing the arm 30 as transformed in a required shape, a spring-loaded part 23 screwed onto the handle 22, and a wire 24 that has one end fixed to the suction attachment 50 side passing through a tube of the arm 30 and the other end fixed to the spring-loaded part 23.

As for the various parts of the main body 20, the grip 21, the handle 22, and the spring-loaded part 23 can be made of resin, as is commonly used in medical instruments, while the wire 24 for example can be made of stainless steel.

As shown in FIG. 1, the arm 30 is connected at one end to the main body 20 and at the other end to the suction attachment 50. The arm 30 is flexible and can be appropriately deformed. The flexibility of the arm 30 requires sufficient rigidity to maintain its shape once deformed, as long as no external force is applied. The arm 30 can be made of stainless steel, aluminum, titanium, or similar materials commonly used for medical instruments.

As shown in FIG. 2A, one end of the tube 40 is connected to the suction attachment 50, and the other end is connected to a vacuum pump (not shown). The tube portion 40 is also flexible. It can be made of polyvinyl chloride (PVC) or similar materials commonly used for medical instruments.

The suction attachment 50 is connected to the arm 30 via a universal joint and is also connected to the tube 40. The suction attachment 50 is characterized by its C-shaped configuration and its flexibility, allowing it to be deformed.

The suction attachment 50 can be sterilized for repeated use or replaced with a new one for each patient as a disposable item.

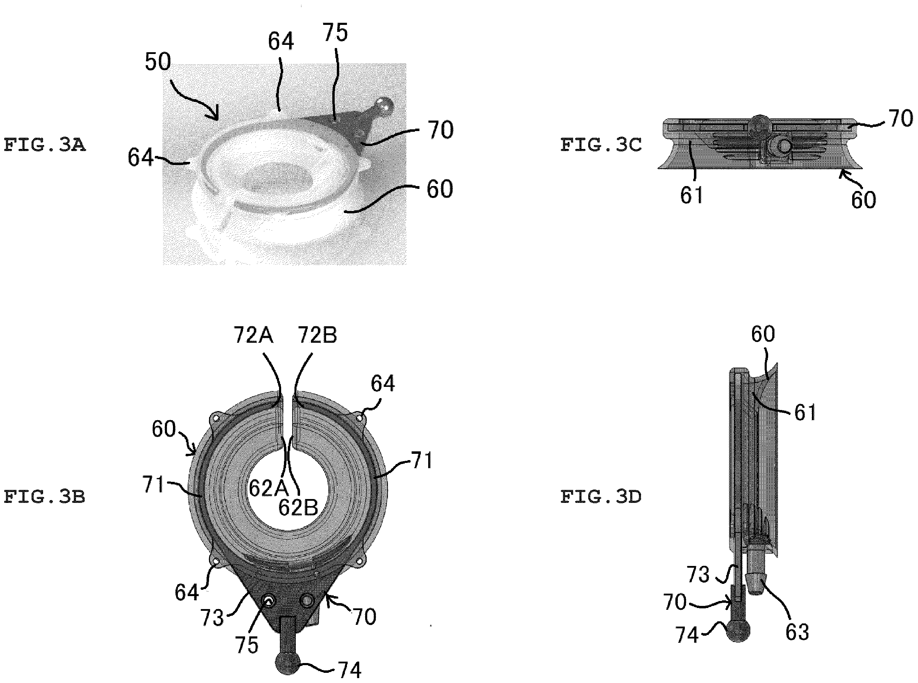

FIG. 3A is a perspective photograph of the suction attachment 50 shown in FIG. 1 and related figures. FIG. 3B is a CAD plan view of the suction attachment 50 shown in FIG. 3A. FIG. 3C is a CAD front view of the suction attachment 50 shown in FIG. 3A. FIG. 3D is a CAD right side view of the suction attachment 50 shown in FIG. 3A.

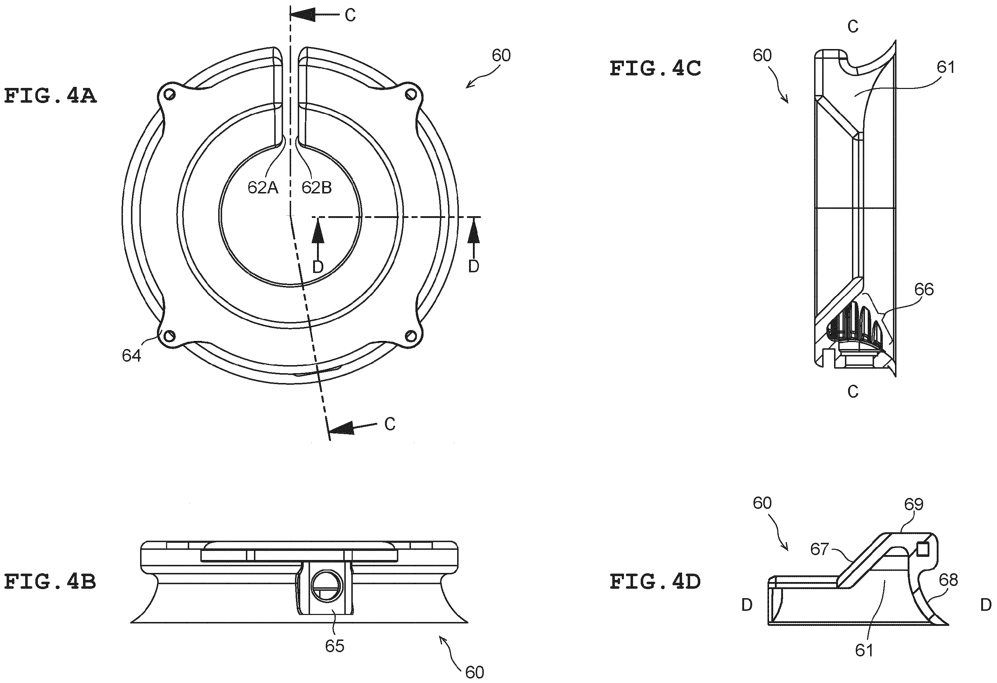

FIG. 4A is a plan view of the flexible channel 60 shown in FIG. 3A. FIG. 4B is a front view of the flexible channel 60 shown in FIG. 4A. FIG. 4C is a cross-sectional view of the flexible channel 60 along line C-C in FIG. 4A. FIG. 4D is a cross-sectional view of the flexible channel 60 along line D-D in FIG. 4A.

As shown in FIG. 3A and related figures, the suction attachment 50 is broadly divided into a flexible channel 60, which makes contact with the surface of the heart of the patient, and a flexible plate 70 that supports the shape of the flexible channel 60.

First, the flexible channel 60 will be explained below. The flexible channel 60 is a part that comes into contact with the heart surface of the patient during coronary artery bypass surgery. The flexible channel 60 can be made of materials such as silicone, polyurethane, polyvinyl chloride, or polycarbonate. Either or both the inner and outer surfaces of the flexible channel 60 can be treated with surface texturing or other surface modifications as needed.

The flexible channel 60 benefits from surface texturing or similar surface treatments in the following ways: texturing an inner surface to a matte finish provides improved contact with the heart's surface, enhancing grip and reducing slippage and texturing an outer surface to a matte finish also improves handling during the removal of the part from the mold during manufacturing.

A hardness of the flexible channel 60 can vary based on its dimensions, particularly its thickness, but it should be in the range of approximately 50 to 90. As shown such as in FIG. 3B and FIG. 4A, the general shape of the flexible channel 60 is C-shaped.

The dimensions of the flexible channel 60 are not limited to the following, but for example, a length corresponding to the outer diameter can be approximately 4.0 cm to 4.5 cm, a length corresponding to an inner diameter can be approximately 1.5 cm to 2.0 cm, and a height for example can be approximately 0.8 cm to 1.2 cm.

Additionally, since the flexible channel 60 has a C-shaped configuration, there is a gap between one end 62A and the other end 62B. In this embodiment, this gap can be used as a passage for the graft vessel that is anastomosed to the coronary artery.

In other words, the C-shaped configuration of the flexible channel 60 is distinct from a donut shape, as it accommodates the passage of a graft vessel anastomosed to the coronary artery, and it is also different from a horseshoe shape because it does not have relatively large open area.

Therefore, a width of this gap (a distance between one end 62A and the other end 62B) is not limited to specific values, but considering a typical thickness of a graft vessel, which is approximately 0.1 cm to 0.25 cm, it can be in the range of about 0.15 cm to 0.40 cm for example. As a result, the flexible channel 60 has a relatively small open area.

The width of the gap is approximately 3% to 10% relative to an average circumference of the outer and inner peripheries of the flexible channel 60. This can be easily derived considering that a midpoint of the outer diameter equivalent length is 1.75 cm and a midpoint of the inner diameter equivalent length is 1.0 cm.

Accordingly, if the flexible channel 60 is regarded as a donut shape, the width of the above-described gap, ranging from 0.15 cm to 0.40 cm, makes an average value of the outer and inner diameters is 1.375 cm, and multiplying the outer and inner diameters by π gives approximately 4.32 cm of an average circumference of the outer and inner peripheries, resulting in the gap width of approximately 3.4% to 9.3% relative to this average circumference of the outer and inner peripheries.

So far as reviewing the Patent Document 1, there does not seem to be a disclosure of the dimensions of the open in the horseshoe shape. However, so far as reviewing FIG. 4 of the Patent Document 1, the width of the open portion in the horseshoe shape appears to be approximately 15% of the average circumference.

Furthermore, since the flexible channel 60 is flexible, it is possible to widen or narrow the gap between one end 62A and the other end 62B in a plane direction or a shear direction of the flexible channel 60. Therefore, if a graft vessel thicker than usual is used for the surgery, the gap in the flexible channel 60 can be appropriately widened to ensure sufficient passage for the graft vessel after anastomosis to the coronary artery.

Additionally, even without a reason for the use of a graft vessel thicker than usual, a curvature of the surface of the heart varies depending on the location where the graft vessel is to be attached, and therefore, the flexible channel 60 can be deformed in the shear direction according to the specific position on the surface of the heart.

Furthermore, as shown such as in FIG. 3B and FIG. 4A, the flexible channel 60 has, for example, four locking sections 64 at the outer upper corners. The respective locking section 64 can be used to secure surgical threads passed through the pericardium around the heart.

As such, while the dimensions of the openings in the respective locking section 64 are not limited to specific values, the dimensions of the openings are typically in the range of approximately 0.10 cm to 0.80 cm. Additionally, if the physician determines that the locking sections 64 are not needed, the locking sections 64 are designed to be easily cut using general or medical scissors.

Also, as shown such as in FIG. 3C and FIG. 4C, the flexible channel 60 includes a suction flow path 61. The suction flow path 61 is a closed space formed between the flexible channel 60 and the surface of the heart, and its shape is a tunnel configuration with a general C-shape.

As shown such as in FIG. 3D and FIG. 4B, the flexible channel 60 has a connecting part 63 to be attached to the suction port 65, which communicates with the suction flow path 61 in the flexible channel 60. The connecting part 63 has a flared shape at both the tip and the base, and the tip of the connecting part 63 is connected to the tube portion 40, while the base thereof is connected to the suction port 65.

When the flexible channel 60 is in contact with the heart surface of the patient during coronary artery bypass surgery, the flexible channel 60 is designed so that the suction flow path 61 is evacuated by a decompression treatment as applying a pressure of approximately 150 mmHg to 500 mmHg through the tube 40 and a vacuum pump (not shown) connected via the connecting part 63.

The suction flow path 61 can either be a single C-shaped pathway directly communicating 1:1 with the suction port 65 or be divided into N sections with a common pathway for each, allowing the sections to be connected in parallel to the suction port 65 at a ratio of N:1 through the common pathways.

Additionally, regarding the manufacturing conditions for the suction port 65, when a center of the flexible channel 60 in FIG. 4A is regarded as an origin of the suction port 65, it is preferable that an angle between an axis of the suction port 65 extending toward the origin and a center line extending through the gap between one end 62A and the other end 62B toward the origin is approximately 10 degrees.

Furthermore, the suction port 65 can be designed with a two-stage structure where the outer diameter, as viewed from the origin, is narrower, for example, approximately 0.3 cm to 0.4 cm, and the inner diameter is wider, approximately 0.4 cm to 0.6 cm, and a base of the connecting part 63 is positioned in a wider part of the suction port 65 to prevent it from falling out.

Near the suction port 65 in the suction flow path 61, ribs 66 are formed as shown in FIG. 4C. The ribs 66 help prevent the suction port 65 from being blocked by the heart surface of the patient being drawn towards it through suction via the tube 40 and also help to avoid stress concentration near the suction port 65 from the tube 40 side.

The ribs 66 are shown as an example in FIG. 4C consisting of four ribs; however, the number of ribs is not limited to four. In this example, the respective rib has a thickness of approximately 0.1 cm to 0.15 cm and a dimension (a length towards the center of the flexible channel 60) of approximately 0.4 cm to 0.6 cm.

Additionally, as shown in FIG. 4D, the suction flow path 61 includes an inner sloped section 67 with a relatively thick cross-sectional linear shape, an outer sloped section 68 with a relatively thin cross-sectional curved shape, and a main body 69 that connects the inner sloped section 67 and the outer sloped section 68.

The dimensions of the inner sloped section 67 are not limited to the following; however, for example, its thickness can be between 0.15 cm and 0.25 cm, extending at an angle of approximately 40 to 50 degrees relative to the plane of the flexible channel 60; its thickness of the lower surface can be between 0.1 cm and 0.2 cm; its horizontal length can be approximately 0.4 cm to 0.6 cm; and a vertical length can be approximately 0.5 cm to 0.7 cm.

The dimensions of the outer sloped section 68 are not limited to the following; however, for example, its thickness can be between 0.08 cm and 0.15 cm, which is approximately a half of the thickness of the inner sloped section 67, descending a position for example about 0.25 cm to 0.32 cm from the outer peripheral edge of the main body section 69 to approximately half the height of the flexible channel 60 at a center of the thickness to curve outward with a radius of curvature of approximately 0.6 cm to 0.8 cm, resulting in the thickness of the lower surface to be between 0.1 cm and 0.2 cm.

As previously described, since the general shape of the heart is spherical, when the flexible channel 60 is placed in contact with the surface of the heart, the suction flow path 61 is designed such that a closed space is often formed by the inner sloped section 67, the outer sloped section 68, and the surface of the heart, and this closed space then becomes the suction flow path 61.

Therefore, when suction is applied through the tube 40 thereafter, the suction flow path 61 is evacuated, and as a result, the relatively thick inner sloped section 67 deforms slightly into a bow shape, while the relatively thin outer sloped section 68 deforms more significantly into a bow shape.

As a result, the area of the surface of the heart near the suction flow path 61 is drawn towards the suction flow path 61, and thereby causing the surface area of the hart within the C-shaped inner region to become more planar, also reducing the transmission of vibrations from the heartbeat of the patient to the treatment opening.

Even if a gap occurs between the flexible channel 60 and the surface of the heart when the flexible channel 60 and the surface of the heart are in contact, preventing the formation of a closed space, if the gap is narrow, suction will draw the nearby heart surface to fill the gap. On the other hand, if the gap is wide, the flexible channel 60 can be deformed to narrow the gap.

Next, the flexible plate 70 will be explained. The flexible plate 70 can be made, for example, from materials such as stainless steel, aluminum, or titanium. The flexible plate 70 can be manufactured, for example, by machining a stainless steel plate with a thickness of approximately 0.1 cm to 0.2 cm.

The flexible plate 70, as shown such as in FIGS. 3A and 3B, includes: a pair of support sections 71 that form a C-shaped configuration, with one end 72A and the other end 72B; a joint section 74 that provides a universal connection between the suction attachment 50 and the arm 30; a triangular base section 73 that connects the support sections 71 and the joint section 74; and a pair of openings 75 formed in the base section 73.

The gap between one end 72A and the other end 72B of the flexible plate 70 is aligned with the gap between one end 62A and the other end 62B of the flexible channel 60. Therefore, these gaps can be used as a passage for the graft vessel, and both the flexible channel 60 and the flexible plate 70 can be deformed in the plane direction or shear direction.

The dimensions of the support sections 71 are not limited to the following examples; however typically, the external diameter equivalent length can range from approximately 2.8 cm to 4.3 cm, the internal diameter equivalent length can range from approximately 1.3 cm to 1.8 cm, and the width between one end 72A and the other end 72B can range from approximately 0.4 cm to 0.6 cm.

The dimensions of the base section 73 are not limited to the following examples; however typically, the length (vertical length in FIG. 3B) can range from approximately 1.3 cm to 1.8 cm, and the width (horizontal length in FIG. 3B) can range from approximately 2.5 cm to 3.5 cm.

The dimensions of the joint section 74 are not limited to the following examples; however typically, the diameter of the spherical tip can range from approximately 0.4 cm to 0.6 cm, the diameter of the cylindrical part connected to the base section 73 can range from approximately 0.25 cm to 0.3 cm, and the length can range from approximately 0.8 cm to 1.2 cm.

The dimensions of the openings 75 are not limited to the following examples; however typically, the diameter can range from approximately 0.2 cm to 0.4 cm. Openings 75 function as positioning jigs during the manufacturing of the suction attachment 50 and function as insertion ports for the tips of tweezers used to grasp the flexible plate 70 during the use of the suction attachment 50.

The suction attachment 50 can be manufactured either by integrally molding the flexible channel 60 and the flexible plate 70 together, or by producing the flexible channel 60 and the flexible plate 70 as separate parts and assembling the flexible plate 70 onto the flexible channel 60.

In this embodiment, the stabilizer has been described with the assumption of its use in human cardiovascular surgeries; however, the stabilizer can also be applied to coronary bypass surgeries for animals such as dogs, cats, horses, cows, and sheep.

As described above, since the flexible channel is not in a horseshoe shape, the suction attachment 50 of this embodiment can effectively suppresses vibrations caused by the heart's beating during coronary bypass surgery and can avoid issues related to the removal of the graft vessel since the flexible channel is not in a ring-shape.

### [BRIEF EXPLANATION OF THE DRAWING(S)]

FIG. 1 is a perspective view showing the schematic appearance of the stabilizer 100 according to an embodiment of the present invention;
FIG. 2A is a right side view of the stabilizer 100 shown in FIG. 1;
FIG. 2B is a cross-sectional view taken along line A-A of the stabilizer 100 shown in FIG. 2A;
FIG. 2C is a left side view of the stabilizer 100 shown in FIG. 2A;
FIG. 2D is a right side view of the stabilizer 100 shown in FIG. 2A;
FIG. 3A is a perspective photograph of the suction attachment 50 shown such as in FIG. 1;
FIG. 3B is a CAD plan view of the suction attachment 50 shown in FIG. 3A;
FIG. 3C is a CAD front view of the suction attachment 50 shown in FIG. 3A;
FIG. 3D is a CAD right side view of the suction attachment 50 shown in Figure 3A;
FIG. 4A is a plan view of the flexible channel 60 shown in FIG. 3A;
FIG. 4B is a front view of the flexible channel 60 shown in FIG. 4A;
FIG. 4C is a cross-sectional view taken along line C-C of the flexible channel 60 shown in FIG. 4A; and
FIG. 4D is a cross-sectional view taken along line D-D of the flexible channel 60 shown in FIG. 4A.

10: Clamp
20: Main body
21: Gripping section
22: Handle
23: Spring-loaded part
24: Wire
30: Arm
40: Tube
50: Suction attachment
60: Flexible channel
61: Suction passage
62A: One end
62B: Other end
63: Connecting section
64: Locking section
65: Suction port
66: Rib section
67: Inner slanted section
68: Outer slanted section
69: Main body
70: Flexible plate
71: Support section
72A: One end
72B: Other end
73: Base section
74: Joint section
75: Opening
100: Stabilizer

## Claims

1. A suction attachment attached to a stabilizer that suppresses vibrations of a heart surface of a patient undergoing a coronary artery bypass surgery, which comprises a flexible channel that forms a suction flow path together with the heart surface when the suction attachment is brought into contact with the heart surface of the patient, wherein
the flexible channel is configured to have a C-shaped tunnel shape to serve as a passage of a graft vessel.

2. The suction attachment according to claim 1, further comprising a C-shaped flexible plate supporting a shape of the flexible channel, wherein
a gap between one end and the other end of the flexible channel is aligned with a gap between one end and the other end of the flexible plate.

3. The suction attachment according to claim 1, wherein
a width of the gap between one end and the other end of the flexible channel is variable due to a deformation of the flexible channel.

4. The suction attachment according to claim 1, wherein
the flexible channel has a plurality of locking sections on an outer upper corner.

5. The suction flow path according to claim 1, wherein
the suction flow path has a relatively thick, straight cross-sectional inner inclined section and a relatively thin, curved cross-sectional inclined section.

6. The suction attachment according to claim 1, wherein
the flexible channel has a suction port connected to the suction flow path.

7. A stabilizer, wherein
the suction attachment according to any one of claims 1-6 is connected to a main body of the stabilizer using a universal joint.
